# FASCICULE DE BREVET EUROPEEN

(11) **EP 0 931 538 B1**
(45) Date de publication et mention de la délivrance du brevet: **12.06.2002**
(21) Numéro de dépôt: 98402997.5
(22) Date de dépôt: 30.11.1998
(51) Int. Cl.: A61K 7/043

(54) **Composition cosmétique de vernis à base de nitrocellulose contenant un agent stabilisant**
Nagellackzusammensetzung auf Basis von Nitrocellulose enthaltend ein Stabilisierungsmittel
Nail varnish composition based on nitrocellulose containing a stabilizing agent

(30) Priorité: 29.12.1997 FR 9716626
(43) Date de publication de la demande: 28.07.1999
(73) Titulaire: L'OREAL, 75008 Paris (FR)
(72) Inventeur: Ramin, Roland, 75014 Paris (FR); Frankfurt, Chris, Old Bridge, NJ 08857 (US)
(74) Mandataire: Lhoste, Catherine

(56) Documents cités:
- FR-A- 2 679 445
- FR-A- 2 737 114
- US-A- 5 102 654

## Description

La présente invention a pour objet une nouvelle composition cosmétique de vernis à base de nitrocellulose, colorée ou incolore, contenant en plus des ingrédients usuels, une substance instable ayant au moins une fonction amine ou amide et un agent stabilisant de ladite substance. US-A-5,102,654 présente des vernis à base de nitrocellulose pour hydratiser des ongles humains contenant des colorants et des substances traitantes à fonction amine et/ou amide.

On a constaté que les compositions cosmétiques de vernis à base de nitrocellulose, présentaient dans le temps une dégradation de certains colorants et/ou pigments ou encore de certaines substances actives traitantes de la kératine de l'ongle, notamment lorsque ceux-ci possédaient au moins une fonction amine ou amide.

Parmi les colorants à fonction amine ou amide susceptibles de dégradation dans les compositions de vernis à base de nitrocellulose, on peut citer entre autres le D&C Red No. 33, le D&C Green No. 6, le D&C Violet No. 2 et le D&C Green No. 5, et parmi les pigments organiques, les laques d'aluminium de D&C Red No. 33 et la guanine ou 2-amino-6 hydroxypurine, cette dernière étant connue pour conférer une brillance nacrée aux vernis par son indice de réfraction élevé.

Parmi les substances actives traitantes de manière cosmétique de la kératine des ongles à fonction amine ou amide susceptibles de dégradation dans les compositions de vernis à base de nitrocellulose, on peut citer notamment le D-panthénol, le panthénate de calcium et le 2-oléoylamino-octadécane-1,3-diol.

D'autres substances actives destinées au traitement cosmétique des ongles, plus particulièrement chez les enfants, peuvent également être susceptibles de dégradation. Parmi celles-ci, on peut citer le benzoate de N-[2-[(2,6-diméthylphényl)amino]-2-oxoéthyl]-N,N-diéthylbenzèneméthanaminium ou BITREX® connu comme agent traitant de l'onychophagie grâce à son goût très amer.

A la suite d'une sélection, on a constaté que certains composés étaient susceptibles de stabiliser les colorants et/ou pigments ainsi que les substances actives traitantes des ongles, tels que définis précédemment et donc d'éviter une dégradation de la couleur ou une perte d'activité des compositions cosmétiques de vernis à base de nitrocellulose.

La présente invention a donc pour objet à titre de produit industriel nouveau, une composition cosmétique de vernis à base de nitrocellulose contenant dans un système solvant pour vernis, un agent plastifiant, une résine et une substance instable à fonction amine ou amide, ladite composition contenant en outre, en une quantité efficace, au moins un agent stabilisant de ladite substance instable choisi parmi le N-chlorosuccinimide et l'acide borique.

Selon l'invention, le pourcentage en agent stabilisant est généralement compris entre 0,7 et 5 %, mais de préférence entre 2 et 4 % en poids par rapport au poids de la nitrocellulose.

Dans les compositions cosmétiques de vernis selon la présente invention, la nitrocellulose qui constitue la substance filmogène est généralement présente en une proportion comprise entre 5 et 20 %, et de préférence entre 10 et 16 % en poids par rapport au poids total de la composition cosmétique de vernis.

Parmi les nitrocelluloses particulièrement préférées, on peut citer les nitrocelluloses du type "RS" ou "SS" et en particulier la nitrocellulose type ¼ seconde RS, la nitrocellulose type ½ seconde RS, la nitrocellulose type ½ seconde SS et la nitrocellulose type ¾ seconde RS.

Selon l'invention, l'agent plastifiant est présent dans la composition de vernis en une proportion comprise entre 2 et 12 %, et de préférence entre 5 et 10 % en poids par rapport au poids total de la composition cosmétique de vernis.

Les agents plastifiants permettent de régler la flexibilité du film sans affaiblir sa résistance ou sa force physique.

Parmi les agents plastifiants susceptibles être utilisés dans les compositions cosmétiques de vernis selon l'invention, on peut citer : le phosphate de tricrésyle, le benzoate de benzyle, le phosphate de tributyle, l'acétyl-ricinoléate de butyle, l'acétyl-ricinoléate de glycéryle, le phtalate de dibutyle, le glycolate de butyle, le phtalate de dioctyle, le stéarate de butyle, le phosphate de tributoxy-éthyle, le phosphate de triphényle, le citrate de triéthyle, le citrate de tributyle, l'acétylcitrate de tributyle, l'acétyl-citrate de triéthyl-2 hexyle, le tartrate de dibutyle, le phtalate de diméthoxyéthyle, le phtalate de di-isobutyle, le phtalate de diamyle, le camphre, le triacétate de glycérol et leurs mélanges.

Selon l'invention, les compositions cosmétiques de vernis contiennent également une résine généralement présente en une proportion comprise entre 0,5 et 15 %, et de préférence entre 5 et 10 % en poids par rapport au poids total de la composition cosmétique de vernis.

Parmi les nombreuses résines utilisables, on préfère employer selon l'invention, des résines du type aryl-sulfonamide formaldéhyde ou aryl-sulfonamide époxy, notamment la résine toluène sulfonamide formaldéhyde plus connue sous les dénominations commerciales de "SANTOLITE MHP®", "SANTOLITE MS 80 %®", et "KETJENFLEX MS 80®", ou encore des résines alkydes telles que celles vendues par la Société DAINIPPON sous la dénomination de "BECKOSOL ODE 230-70®".

Ces résines, tout en augmentant le pouvoir filmogène, améliorent le brillant ainsi que l'adhérence.

Selon l'invention, le système solvant de la composition de vernis est présent en une proportion comprise entre 55 et 90 % en poids par rapport au poids total du vernis.

Ce système solvant est essentiellement constitué par un mélange de divers solvants organiques volatils, ceci en vue d'obtenir des temps de séchage relativement courts.

Parmi ces solvants on peut citer l'acétate de méthyle, l'acétate d'éthyle, l'acétate d'isopropyle, l'acétate de butyle, l'acétate d'amyle, l'acétate de méthoxy-2 éthyle, l'acétone, la méthyl-éthyl cétone et la méthyl-isobutyl cétone.

Le système solvant peut comprendre également un diluant qui est de préférence un hydrocarbure, linéaire ou ramifié, saturé, tel que l'hexane ou l'octane, ou encore un hydrocarbure aromatique tel que le toluène ou le xylène, dans une proportion généralement comprise entre 10 et 35 % par rapport au poids total de la composition cosmétique de vernis.

Le système solvant peut également inclure d'autres solvants volatils tels que de l'éthanol, du n-propanol, de l'isopropanol, du n-butanol ou leurs mélanges.

Lorsque les compositions cosmétiques de vernis selon l'invention sont colorées, elles peuvent contenir en outre d'autres colorants et/ou pigments de nature organique ou inorganique.

Parmi les pigments organiques, on peut citer les D&C Red No. 10, 11, 12 et 13, le D&C Red No. 7, les D&C Red No. 5 et 6 et les D&C Red No. 34, ainsi que les laques telles que la laque D&C Yellow No. 5 et la laque D&C Red No. 2.

Parmi les pigments inorganiques, on peut citer le dioxyde de titane, l'oxychlorure de bismuth, l'oxyde de fer brun et les oxydes de fer rouge.

D'une façon générale, les colorants et/ou pigments sont présents dans les compositions cosmétiques de vernis selon l'invention, en une proportion comprise entre 0,01 et 2 % en poids par rapport au poids total de la composition de vernis.

En vue d'éviter la sédimentation des pigments, certains agents thixotropes peuvent être employés, tels que par exemple la "BENTONE 27®" ou la "BENTONE 38®".

Les compositions cosmétiques de vernis selon la présente invention peuvent également contenir des adjuvants conventionnels tels que des filtres UVA et/ou UVB, qui sont des dérivés de benzophénone et le 2-cyano-3,3-diphényl acrylate d'éthyle, des agents dispersants et mouillants, des agents matifiants, des agents d'adhérence, des agents d'étalement, des agents rhéologiques tels que les silices pyrogénées, des antioxydants, des conservateurs, des épaississants et des agents durcisseurs de l'ongle.

On va maintenant donner à titre d'illustration, plusieurs exemples de compositions de vernis selon l'invention.

### EXEMPLES DE VERNIS

### EXEMPLE 1 : Vernis à ongles coloré

- Nitrocellulose 20 g
- Acétylcitrate de tributyle 6 g
- Résine toluène sulfonamide formaldéhyde 4 g
- N-chlorosuccinimide 0,4 g
- D&C Red No. 33 0,001 g
- Autres pigments et colorants 1 g
- (Acétate d'éthyle, acétate de butyle,
   acétone 20/60/20) q.s.p. 100 g

Ce vernis présente une bonne stabilité de la coloration au cours du temps.

En l'absence de N-chlorosuccinimide, on observe une modification rapide de la coloration du vernis.

### EXEMPLE 2 : Vernis pour le soin des ongles

- Nitrocellulose 20 g
- Acétylcitrate de tributyle 6 g
- Résine toluène sulfonamide formaldéhyde 4 g
- Acide borique (en solution aqueuse à 10 %) 0,4 g
- Argile (Bentone 27®) 1 g
- Alcool isopropylique 6 g
- Pigments organiques 1 g
- D-panthénol 0,5 g
- (Acétate d'éthyle,
   acétate de butyle 50/50) q.s.p. 100 g

En l'absence de l'acide borique, l'analyse de ce vernis montre une forte dégradation du D-panthénol.

## Revendications

1. Composition cosmétique de vernis à base de nitrocellulose contenant dans un système solvant pour vernis, au moins un agent plastifiant, au moins une résine et au moins une substance instable à fonction amine ou amide, **caractérisée en ce qu'**elle contient en outre, en une quantité efficace, au moins un agent stabilisant de ladite substance instable choisi parmi le N-chlorosuccinimide et l'acide borique.

2. Composition selon la revendication 1, **caractérisée en ce que** la nitrocellulose est présente en une proportion comprise entre 5 et 20 %, en poids par rapport au poids total de la composition.

3. Composition selon les revendications 1 et 2, **caractérisée en ce que** ledit agent stabilisant est présent en une proportion comprise entre 0,7 et 5 % en poids par rapport au poids de la nitrocellulose.

4. Composition selon l'une quelconque des revendications précédentes, **caractérisée en ce que** la substance instable est un pigment organique choisi dans le groupe constitué par le D&C Red No. 33, le D&C Green No. 6, le D&C Violet No. 2, le D&C Green No. 5, la laque d'aluminium du R&C Red No. 33 et la guanine.

5. Composition selon l'une quelconque des revendications 1 à 3, **caractérisée en ce que** la substance instable est une substance active traitante choisie dans le groupe constitué par le D-panthénol, le panthénate de calcium et le 2-oléoylamino-octadécane-1,3-diol.

6. Composition selon l'une quelconque des revendications 1 à 3, **caractérisée en ce que** la substance instable est le benzbate de N-[2-[(2,6-diméthylphényl)amino]-2-oxoéthyl]-N,N-diéthylben-zèneméthanaminium.

7. Composition selon l'une quelconque des revendications précédentes, **caractérisée en ce que** le système solvant est présent en une proportion comprise entre 55 et 90 % en poids par rapport au poids total de la composition.

8. Composition selon l'une quelconque des revendications précédentes, **caractérisée en ce que** la résine est présente en une proportion comprise entre 0,5 et 15 % en poids par rapport au poids total de la composition.

9. Composition selon l'une quelconque des revendications précédentes, **caractérisée en ce que** l'agent plastifiant est présent en une proportion comprise entre 2 et 12 % en poids par rapport au poids total de la composition.

10. Composition selon l'une quelconque des revendications précédentes, **caractérisée en ce qu'**elle contient en outre au moins un adjuvant conventionnel choisi dans le groupe constitué par les agents thixotropes, les filtres solaires UVA et/ou UVB, les agents dispersants et mouillants, les agents matifiants, les agents d'adhérence, les agents d'étalement, les agents rhéologiques, les conservateurs, les antioxydants, les épaississants et les agents durcisseurs de l'ongle.

11. Procédé de maquillage et/ou de traitement cosmétique des ongles, **caractérisé en ce que** l'on applique sur les ongles, une composition selon l'une quelconque des revendications 1 à 10.

12. Utilisation dans une composition cosmétique de vernis à base de nitroceliulose, d'au moins un composé choisi parmi le N-chlorosuccinimide et l'acide borique en tant qu'agent stabilisant d'au moins une substance instable à fonction amine ou amide.

## Claims

1. Nitrocellulose-based cosmetic varnish composition comprising within a varnish solvent system at least one plasticizer, at least one resin and at least one unstable amino- or amido-functional substance, **characterized in that** the said composition additionally comprises, in a quantity effective for stabilizing the said unstable substance, at least one stabilizer selected from N-chlorosuccinimide and boric acid.

2. Composition according to Claim 1, **characterized in that** the nitrocellulose is present in a proportion of between 5 and 20% by weight relative to the total weight of the composition.

3. Composition according to Claims 1 and 2, **characterized in that** the said stabilizer is present in a proportion of between 0.7 and 5% by weight relative to the weight of nitrocellulose.

4. Composition according to any one of the preceding claims, **characterized in that** the unstable substance is an organic pigment selected from the group consisting of D&C Red No. 33, D&C Green No. 6, D&C Violet No. 2, D&C Green No. 5, D&C Red No. 33 aluminium lake, and guanine.

5. Composition according to any one of Claims 1 to 3, **characterized in that** the unstable substance is an active treatment substance selected from the group consisting of D-panthenol, calcium panthenate and 2-oleoylaminooctadecane-1,3-diol.

6. Composition according to any one of Claims 1 to 3, **characterized in that** the unstable substance is N-[2-[(2,6-dimethylphenyl)amino]-2-oxoethyl]-N,N-diethylbenzenemethanaminium benzoate.

7. Composition according to any one of the preceding claims, **characterized in that** the solvent system is present in a proportion of between 55 and 90% by weight relative to the total weight of the composition.

8. Composition according to any one of the preceding claims, **characterized in that** the resin is present in a proportion of between 0.5 and 15% by weight relative to the total weight of the composition.

9. Composition according to any one of the preceding claims, **characterized in that** the plasticizer is present in a proportion of between 2 and 12% by weight relative to the total weight of the composition.

10. Composition according to any one of the preceding claims, **characterized in that** it additionally comprises at least one conventional adjuvant selected from the group consisting of thixotropic agents, UVA and/or UVB sunscreens, dispersants and wetting agents, dulling agents, adhesion agents, spreading agents, rheological agents, preservatives, antioxidants, thickeners and nail hardeners.

11. Method of decorating and/or cosmetically treating the nails, **characterized in that** a composition according to any one of Claims 1 to 10 is applied to the nails.

12. Use in a nitrocellulose-based cosmetic varnish composition of at least one compound selected from N-chlorosuccinimide and boric acid as a stabilizer for at least one unstable amino- or amido-functional substance.

## Patentansprüche

1. Kosmetische Lackzusammensetzung auf Nitrocellulosebasis, die in einem Lösungsmittelsystem für Lacke mindestens einen Weichmacher, mindestens ein Harz und mindestens eine instabile Substanz mit Amino- oder Amidgruppe enthält, **dadurch gekennzeichnet, daß** sie ferner in einer wirksamen Menge mindestens ein Mittel zur Stabilisierung der instabilen Substanz enthält, das unter N-Chlorsuccinimid und Borsäure ausgewählt ist.

2. Zusammensetzung nach Anspruch 1, **dadurch gekennzeichnet, daß** die Nitrocellulose in einem Mengenanteil von 5 bis 20 Gew.-%, bezogen auf das Gesamtgewicht der Zusammensetzung, vorliegt.

3. Zusammensetzung nach Anspruch 1 oder 2, **dadurch gekennzeichnet, daß** das Stabilisierungsmittel in einem Mengenanteil von 0,7 bis 5 Gew.-%, bezogen auf das Gewicht der Nitrocellulose, vorliegt.

4. Zusammensetzung nach einem der vorhergehenden Ansprüche, **dadurch gekennzeichnet, daß** die instabile Substanz ein organisches Pigment ist, das unter D&C Red Nr. 33, D&C Green Nr. 6, D&C Violet Nr. 2, D&C Green Nr. 5, dem Aluminiumlack von D&C Red Nr. 33 und Guanin ausgewählt ist.

5. Zusammensetzung nach einem der Ansprüche 1 bis 3, **dadurch gekennzeichnet, daß** die instabile Substanz ein Wirkstoff zur Behandlung ist, der unter D-Panthenol, Calciumpanthenat und 2-Oleoylamino-octadecan-1,3-diol ausgewählt ist.

6. Zusammensetzung nach einem der Ansprüche 1 bis 3, **dadurch gekennzeichnet, daß** die instabile Substanz das N-[2-[(2,6-Dimethylphenyl)amino]-2-oxoethyl]-N,N-diethylbenzolmethanaminiumbenzoat ist.

7. Zusammensetzung nach einem der vorhergehenden Ansprüche, **dadurch gekennzeichnet, daß** das Lösungsmittelsystem in einem Mengenanteil von 55 bis 90 Gew.-%, bezogen auf das Gesamtgewicht der Zusammensetzung, vorliegt.

8. Zusammensetzung nach einem der vorhergehenden Ansprüche, **dadurch gekennzeichnet, daß** das Harz in einem Mengenanteil von 0,5 bis 15 Gew.-%, bezogen auf das Gesamtgewicht der Zusammensetzung, vorliegt.

9. Zusammensetzung nach einem der vorhergehenden Ansprüche, **dadurch gekennzeichnet, daß** der Weichmacher in einem Mengenanteil von 2 bis 12 Gew.-%, bezogen auf das Gesamtgewicht der Zusammensetzung, vorliegt.

10. Zusammensetzung nach einem der vorhergehenden Ansprüche, **dadurch gekennzeichnet, daß** sie ferner mindestens einen herkömmlichen Zusatzstoff enthält, der unter den Thixotropiermitteln, UV-Aund/oder UV-B-Filtern, Dispergiermitteln, Netzmitteln, Mattierungsmitteln, Haftmitteln, Spreitmitteln, Rheologiehilfsmitteln, Konservierungsmitteln, Antioxidantien, Verdickungsmitteln und Nagelhärtern ausgewählt ist.

11. Verfahren zum Schminken und/oder zur kosmetischen Behandlung der Nägel, **dadurch gekennzeichnet, daß** auf die Nägel eine Zusammensetzung nach einem der Ansprüche 1 bis 10 aufgetragen wird.

12. Verwendung mindestens einer Verbindung, die unter N-Chlorsuccinimid und Borsäure ausgewählt ist, als Mittel zur Stabilisierung mindestens einer instabilen Substanz mit Amino- oder Amidgruppe in einer kosmetischen Lackzusammensetzung auf Nitrocellulosebasis.
